# EUROPEAN PATENT SPECIFICATION

(11) **EP 2 683 312 B1**
(45) Date of publication and mention of the grant of the patent: **13.07.2016**
(21) Application number: 12715162.9
(22) Date of filing: 09.03.2012
(51) Int. Cl.: A61B 17/72, A61B 17/58

(54) **DISPOSABLE DEVICE FOR TREATMENT OF INFECTIONS OF HUMAN LIMBS AND METHOD FOR ITS REALISATION**
EINWEG-VORRICHTUNG ZUR BEHANDLUNG VON INFEKTIONEN DER MENSCHLICHEN EXTREMITÄTEN UND VERFAHREN ZU IHRER HERSTELLUNG
DISPOSITIF JETABLE POUR LE TRAITEMENT D'INFECTIONS DE MEMBRES HUMAINS ET SON PROCÉDÉ DE RÉALISATION

(30) Priority: 10.03.2011 IT VR20110051
(43) Date of publication of application: 15.01.2014
(73) Proprietor: Tecres S.P.A., 37066 Sommacampagna (VR) (IT)
(72) Inventor: SOFFIATTI, Renzo, I-37066 Sommacampagna (Verona) (IT); FACCIOLI, Giovanni, I-37066 Sommacampagna (Verona) (IT)
(74) Representative: Feltrinelli, Secondo Andrea
(86) International application number: PCT/IB2012/051126
(87) International publication number: WO 2012/120480

(56) References cited:
- WO-A1-98/19617
- WO-A1-2006/090226
- US-A- 5 618 286
- US-A1- 2007 202 342
- US-A1- 2009 104 095

## Description

### TECHNICAL FIELD OF THE INVENTION

The present invention relates to a device usable in the medical field, in the technical field of surgical instruments, and in particular its object is a temporary disposable device for preventing and treating infections of the human body limbs, in particular of limbs with long bones capable of being stabilised by means of intramedullary nails. Intramedullary nails or rods are used for aligning and stabilising fractures and are longitudinally inserted into the medullary canal located in the middle of the limbs' long bones (for example femur or tibia). The present invention also relates to a method for making such device.

### PRIOR ART

In order to stabilise the long bones of the human body subsequent to fractures, malformations or similar pathological situations, it is known that known devices are used, such as intramedullary nails, consisting of hollow or solid and more or less curved metal rods which are inserted within the intramedullary cavity of the bones to be stabilised and are anchored to the stumps or parts to be stabilised by means of pins or cross screws, generally in the distal or proximal zone of the limb. Once the fracture has been stabilised and healed, the pins are removed and the nail is extracted from the medullary canal.

In addition to the invasiveness of the implantation operation, a problem related to such treatment is the onset of infection hotbeds which may develop locally due to bacteria that normally come into contact with the implantation site and are propagated by the presence of the metal synthesis medium in contact with the bony tissue.

The International application WO98/19617 discloses an orthopedic implant comprising a thermoplastic polymer or a composite comprising, in one embodiment, polyetheretherketone reinforced with 10 % by volume of glass fibers, with an elastic modulus approximating the elastic modulus of bone. A porous coating is formed on the implant surface by creating a roughness thereon, by coating the surface with hydroxyapatite or by embedding a biocompatible material such as titanium in the surface. A two piece embodiment of the implant is joined and locked together, after the opposite ends of each piece are inserted in the medullary canal, using an interlocking mechanism comprising a fluted protrusion on one piece and a corresponding fluted cavity in the other piece with the fluted portions being complementarily tapered.

In order to minimise such drawbacks, it is absolutely advisable for the implantation of the above devices to take place in a totally sterile manner and for the concerned part to be treated with substances adapted to prevent and/or treat such infections.

In an attempt to overcome, at least partly, the drawbacks mentioned above, solutions have been developed that allow the application of such substances adapted to prevent and/or treat the above infections, directly in situ, as they are or admixed with bone cement.

Patent US 4863444 describes a rod-shaped carrier wherethrough an antibiotic may be delivered. Due to the elongated configuration thereof, the rod may be inserted within a channel made through the skin and the soft tissues for the application of an outer device comprising a screw or a nail introduced crosswise into the bone.

A solution is also known which provides for making a coating for the stabilising device by the use of moulds to be used at the workshop or directly during the surgery right before implanting the same device. Such solution exhibits some known drawbacks. Firstly, it is substantially of the handicraft type, with consequent necessary expertise of the operator that must be necessarily skilled. Such a way of operating, moreover, implies a large production of processing scrap, with consequent waste of materials and increase in the overall operation cost.

In this way, moreover, the coated device is exposed for too long to the air and to the contact with foreign bodies, with consequent high danger of attack by bacteria and contaminating agents. Moreover the coated device exiting from the mould exhibits a large amount of burrs along the longitudinal development thereof, which must be removed for allowing the proper positioning of the same device. This implies a considerable extension of the implantation time with consequent discomfort for the patient.

A further aspect of the invention relates to the need of temporarily replacing an intramedullary nail for various reasons, such as for example a relapse or a dislodgement of the implant, normally accompanied by site infections.

In such situations it is necessary to remove the pre-existing intramedullary nail and treat the implantation zone before setting the new nail. Meanwhile it is necessary to keep the site geometry unchanged to prevent the tissue shortening and deformation, by a temporary spacing device which is at the same time capable of preventing and/or healing the infections related to the old and new implant.

### OBJECTS OF INVENTION

The object of the present invention is to overcome the above drawbacks by providing a temporary device for treating infections which has high efficiency and inexpensiveness features.

A particular object is to provide a ready-to-use temporary device for treating infections which prevents the use of skilled staff.

A further object is to provide a temporary device for treating infections which allows the time of exposure to the environment to be reduced and ensure high sterility.

Another object is to provide a temporary device for treating infections which allows the maximum rationalisation of the use of materials and products available on the market.

Such objects as well as others that will appear more clearly hereinafter are achieved by a temporary disposable device according to claim 1.

A particular object is to provide a method for making a ready-to-use temporary device for treating infections which prevents the use of skilled staff. A further object is to provide a method for making a temporary device for treating infections which allows the time of exposure to the environment to be reduced and ensure high sterility.

Another object is to provide a method for making a temporary device for treating infections which allows the maximum rationalisation of the use of materials and products available on the market.

Such objects as well as others that will appear more clearly hereinafter are achieved by a method for making a disposable device according to claim 15.

This particular configuration of the invention allows a ready-to-use temporary device for treating infections to be provided, making the presence of skilled staff unnecessary for making the same. Moreover, this particular embodiment according to the invention allows the use of materials to be optimised, totally eliminating processing scrap and reducing the time needed for inserting the same nail.

Moreover, the temporary device for treating infections is made of a material biologically compatible with the limbs to be treated.

Moreover, the temporary device according to the invention allows the maximum rationalisation of the materials and products available on the market, making the make thereof even more inexpensive.

This particular aspect of the invention provides a temporary device for treating infections that ensures a high sterility.

### BRIEF DESCRIPTION OF THE DRAWINGS

These and further advantages shall be better understood by any man skilled in the art from the following description and annexed drawings, provided by way of a non limiting example, wherein:

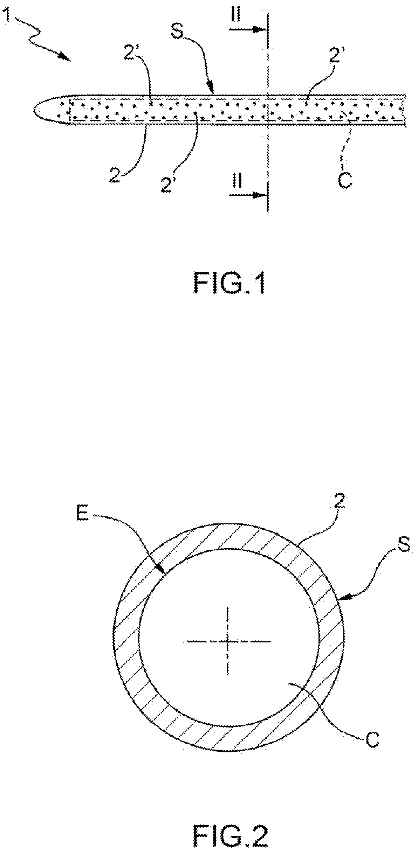

FIG. 1 shows a side view of an embodiment of the temporary device according to the invention;
FIG. 2 shows a sectional view of the temporary device of FIG. 1 taken along a trace plane II-II;
FIG. 3 shows an axonometric view of another configuration of the temporary device according to the invention;
FIG. 4 shows a view of a detail of the temporary device of FIG. 3;
FIG. 5 shows a schematic view of a partial section of a version of the present invention;
FIG. 6 shows an exemplary view at the optical microscope of the present invention obtained with a 20X magnification.

### EMBODIMENTS OF THE INVENTION

With reference to the above figures, the device according to the invention globally indicated with reference numeral 1, is of the disposable and temporary type and will in particular be intended to stabilise limbs with long bones by means of intramedullary nails. In one embodiment described by way of a non limiting example hereinafter, device 1 comprises a rod-shaped intramedullary nail C of known type. Device 1 may further comprise other types of devices available on the market and adapted to be used as stabilisers of limbs with long bones. Device 1 comprises an outer surface E.

In contact with at least one portion of the outer surface E, device 1 comprises a surface layer 2 comprising a biologically compatible material in a solidifiable fluid form.

In one version of the invention, the material the surface layer 2 is made of is preferably selected from bone cements, which may also be of the absorbable or biodegradable type.

The material the surface layer 2 is made of may be insoluble in the human body and therefore permanent, or soluble and therefore it can be reabsorbed in the human body.

The material insoluble in the human body may be obtained from a dispersion of polymethyl methacrylate polymers (PMMA) and/or PMMA esters, such as butyl methacrylate, etcetera, and/or copolymers, such as PMMA and polymethyl styrene copolymer, etcetera, in an organic solvent such as for example methyl methacrylate (MMA), chloroform or other organic solvents and/or water.

On the other hand, the soluble material may be obtained from a dispersion of polymers soluble in the human body, such as polyglycolic acid (PGA), polylactic acid (PLA), polyglycolic acid and polylactic acid copolymers (PGA-PLA), L-polylactic acid and polyglycolic acid copolymers (PLLA co PGA), L,D-polylactic acid and trimethylene carbonate copolymers (PLDA co TMC), polyvinylpyrrolidone (PVP) derivatives, polyvinyl chloride (PVC), amide or cellulose derivatives, etcetera and an organic solvent such as methyl methacrylate, chloroform or other organic solvents suitable for dissolving the above polymers and/or water.

Possible drugs and/or medications may be added to the material making up the surface layer 2.

The material that is soluble or insoluble in the human body may be dispersed or solubilised in the special organic and/or aqueous solvents indicated above and thereafter be applied onto and/or placed in contact with the intramedullary nail C or with device 1.

The above materials comprise all the optically active isomers and/or the racemic forms of the same. The surface layer 2 strictly and continuously adheres to the outer surface E of device 1, as will be better described hereinafter.

Therefore, once the surface layer 2 has been applied to device 1, it is not possible anymore to separate it from the latter.

A perfect and steady adhesion is thus obtained between the surface layer 2 and device 1 whereon it is applied, ensuring a stability of the same device in the performance as stabiliser of fractures of the human body bones. Once the material it is made of has cured and solidified, the surface layer 2 comprises a solid porous or spongy material.

The surface layer 2 comprises drugs and/or medications in dry form in order to be mixed with said material and/or in fluid form in order to allow the impregnation of the surface layer 2 beforehand or upon the insertion within the stabilisation site. In fact, according to the invention, the surface layer 2 is provided with pores 2' for the impregnation with drugs and/or medications for treating infections, such as antibiotics, growth promoters, anti-inflammatory and/or anti-tumour substances.

In the present description, the term "pores" indicates: a small space or a distance between the molecules making up a body, in particular of the surface layer 2. Pores 2' are arbitrarily provided on the outer surface S and within the surface layer 2. Pores 2' are mostly interconnected and have a random arrangement. Pores 2' are natural and have micrometric dimensions.

Pores 2' are preferably non-through pores, that is, they do not form a direct and free path between the outer surface S and the inner one of the surface layer 2.

Pores 2' have dimensions of less than 100 microns for preventing the bone growth through the same.

Such aspect is very important in view of the temporary nature of device 1. In fact, by preventing the migration and growth of the bone tissue cells therein, pores 2' of dimensions of less than 100 microns allow a better removability of the same device 1.

Pores 2' and/or the surface layer 2 through pores 2' may be impregnated with drugs and/or medications for treating infections. Pores 2' allow containing the drugs and/or medications within the structure of the surface layer 2 and releasing such substances to the external environment, for example the bone. Pores 2' are not in direct communication with the intramedullary nail C or with device 1. Conversely, pores 2' are locked or sealed by the intramedullary nail C provided within the surface layer 2 or by the same device 1. Pores 2' act as a sort of "small containers" for containing the above drugs and/or medications which are then released over time when they contact the implantation site to be treated.

If the drugs and/or medications are in dry form, such substances are directly contained within the surface layer 2 and pores 2' provide a path for the release of the same from the surface layer 2 outwards.

As an alternative, the surface layer 2 does not directly contain the above drugs and/or medications but being porous or spongy thanks to pores 2', it is suitable impregnated by these substances present in fluid form. Due to the fact that the surface layer 2 is impregnated with such drugs and/or medications through pores 2' distributed on the outer surface S thereof and within the surface layer 2, the impregnation time may be controlled. For example, the impregnation may take place at different times according to the specific requirements, such as for example beforehand, before or during the insertion in a stabilisation site, or even before the packaging of device 1.

The surface layer 2 has different thicknesses according to the thickness of the material applied and to the number of applications of the same. In particular, the surface layer 2 has a thickness variable between 10 microns and 1000 microns. When the thickness of the surface layer 2 is about 10 microns, it forms a sort of barrier against the bone growth. This is particularly useful in the case of nails made of titanium - more than hard steel AISI 316L or cobalt-chromium alloys - since the surface layer 2 creates a barrier for preventing the strict trapping of the bony tissue onto the titanium nail, making the subsequent removal thereof easier.

The thickness of the surface layer is about 100-400 microns to be useful in the release of drugs or other medications; if the thickness exceeds 600 microns, the drug release time increases according to the larger amount of drugs or other medications incorporated within the surface layer 2.

In any case, if the thickness of the surface layer 2 exceeds 250 microns, the size of the intramedullary nail or of device 1 varies, therefore in one version of the invention this should be avoided.

The thickness of the surface layer 2 is homogeneous; the surface layer 2 exhibits no application discontinuity on device 1; moreover, the surface layer 2 affects the whole surface E of device 1 with which the material of the surface layer 2 comes into contact, as better explained hereinafter.

The homogeneous thickness of the surface layer 2 may be obtained both if the outer surface E of device 1 is substantially linear and if device 1 comprises slits, grooves, recesses or other similar configurations.

The fact that the thickness of the surface layer 2 is homogeneous and that the latter affects the whole surface of device 1 with which it comes into contact allows a better distribution of the substances contained within the same surface layer 2 at a spatial level.

If the thickness and the arrangement of the surface layer 2 were not homogeneous, in fact, in some contact zones with the bone there may be the risk of an excessive concentration of substances contained in the surface layer 2 and in some others, a complete absence of the same.

Moreover, thanks to the thickness homogeneity and to the application without discontinuity of the surface layer 2, zones of device 1 are prevented from being without surface layer 2. In this way, the material of device 1 is prevented from directly contacting the bony tissue since otherwise the prevention and/or healing of the infection in such zones would not take place.

According to one version of the invention, the intramedullary nail C may be made of a relatively stiff metal or non metal material so as to define an actual spacing device as a whole.

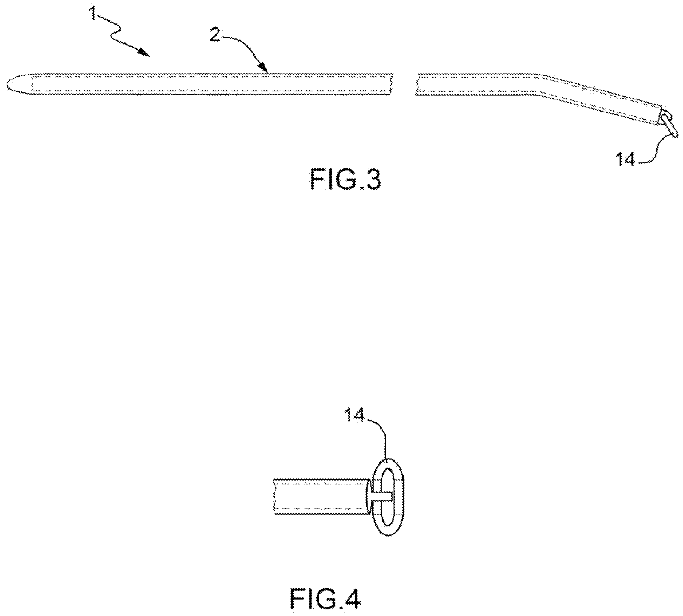

Moreover, as shown in figures 3 and 4, a gripping element 14, such as a ring, may be attached to the proximal end of the intramedullary nail C to aid in the removal of device 1.

Also in this case, the surface layer 2 may contain drugs and/or medications for treating the infections, such as antibiotics, growth promoters, anti-inflammatory and/or anti-tumour substances. In the case of drugs and/or medications in fluid form, the impregnation may take place beforehand, prior to the packaging of the device, or upon the insertion thereof into the site by the surgeon, as mentioned above.

In one version of the invention, device 1 comprises a sterile enclosure or blister so as to form an assembly for treating infections of the human body limbs, in particular limbs with long bones sensitive to the stabilisation with intramedullary nail, and for ensuring the utmost sterility conditions and guarantee of storage prior to the installation.

This particular configuration of the invention provides a ready-to-use temporary device for treating infections which eliminates the need of using skilled staff. Moreover, the device optimises the use of materials and products currently available on the market, considerably reducing processing scrap.

Finally, such particular configuration provides a temporary device 1 for treating infections which allows reducing the exposure time of the same to the environment and ensures the best sterility conditions.

The invention further comprises a method for making a temporary disposable device for treating infections of the human body limbs comprising the steps of providing a device 1 suitable for the stabilisation of human body limbs and provided with an outer surface E, preparing a solution comprising a biologically compatible material in solidifiable fluid form with an organic and/or aqueous solvent, applying the solution or suspension on at least a portion of the outer surface E of device 1 according to a continuous and homogeneous arrangement on the portion of the outer surface (E) it comes into contact with, drying device 1 thus coated for fully eliminating the residual solvent through the evaporation of the same and/or the material curing, thus forming a porous or spongy surface layer 2 provided with an outer surface S and comprising pores 2' arbitrarily provided within the outer surface S or within the surface layer 2 and having a random arrangement, wherein the surface layer 2 is strictly adhering to device 1.

The method according to the present invention comprises a step of preparing a substantially rod-shaped intramedullary nail C.

The method according to the present invention comprises a step of preparing a solution wherein the biologically compatible material in solidifiable fluid form exhibits a concentration in the solvent of about 1-75% (w/w).

The method according to the present invention comprises a step of homogenising the solution of biologically compatible material prior to the step of application of the same.

The method according to the present invention further comprises a step of waiting 10-60 minutes after the application step.

The method according to the present invention comprises a step of repeating the application step and the waiting step at least two more times.

The method according to the present invention comprises a step of evaporating the solvent for eliminating most of the same solvent after the application step or the waiting step.

The evaporation step is carried out to the air; the evaporation step is carried out for about 24 hours. The drying step is carried out for about 5 hours; the drying step is carried out at temperature of about 70 °C.

The surface layer 2 adheres to device 1 or to the intramedullary nail C due to the adhesion that is obtained between the material that makes up the surface layer 2 and device 1 or the intramedullary nail C. The nature of such adhesion may be of the mechanical type and of the chemical-physical type. An example of mechanical adhesion is caused by irregularities present on the outer surface E of device 1 or of the intramedullary nail C, such as for example undercuts and/or micro-cavities wherein the material that makes up the surface layer 2 roots. An example of chemical-physical adhesion, on the other hand, is caused by the attraction induced by weak forces of the molecular type, such as for example Van der Waals forces, which are exerted between the materials that make up the surface layer 2 and device 1 or the same intramedullary nail C. The application step is carried out through at least one immersion of device 1 or of the intramedullary nail C in the solution and/or through coating and/or spraying of the same solution on device 1 or on the intramedullary nail C.

The method further comprises the steps of adding a drug or other medication to the solution, such as for example an antibiotic, at a concentration in the material of about 5-65% (w/w), and/or impregnating the surface layer 2 with drugs and/or medications for treating infections, beforehand or upon the insertion within the stabilisation site.

In one version of the invention, the immersion speed of the intramedullary nail C in the solution is in the range between 1 and 60 cm/min and is preferably fast: equal to about 30 cm/min; the extraction speed of the same is in the range between 1 and 60 cm/min and is preferably slow: equal to about 5 cm/min.

Preferably, the concentration of material in the solvent is about 12-38% (w/w) and more preferably about 25% (w/w); preferably, the concentration of antibiotic, for example gentamicin sulphate, in the base material is about 7-23% (w/w) and more preferably about 15%. Preferably, the immersion time is 30 minutes and the number of repetitions is of 3 more times.

During the material curing and solidification, the solvent, the air and/or the solvent vapours that are a part thereof, evaporate and create the porosity trying to find an escape route. Once the curing has occurred, the surface layer 2 remains on device 1 or on the intramedullary nail C in the form of a thin layer of solid and porous or spongy material, whereon and wherein the forming of pores 2' takes place.

In this version, the surface layer 2 is obtained by the reaction of the above substances and once device 1 has been inserted within the stabilisation site, the reaction of the surface layer 2 has already ended: the material that makes up the surface layer 2 has already cured and is porous or spongy and is therefore used for releasing drugs and/or medications.

Pores 2' are naturally obtained by the evaporation reaction of the solvent and by the curing of the material that makes up the surface layer 2. Thanks to the fact that they are formed naturally during the forming of the surface layer 2, pores 2' do not require a specific making step and this makes the production of device 1 easier, faster and less expensive, unlike known devices that comprise tubular members wherein holes or ducts must be made, which are operatively obtained within the same device.

Likewise, device 1 needs not to be made specifically for the purpose.

In fact, device 1 of the present invention may comprise any device available on the market adapted to the stabilisation of long bones. In this way, such devices available on the market become the temporary disposable device according to the present invention. This is obtained also thanks to the fact that the surface layer 2 adheres to device 1 without the need of a previous preparation of such device and without the need of having particular gripping or roughness features in the outer surface E of device 1.

In one version of the invention, however, it is preferable to use a device 1 having a substantially linear outer surface E, without grooves or recesses with a particular geometry, in order to ensure a better duration of the same device and prevent a possible breakage thereof under stress.

Thanks to the method according to the present invention, a homogeneous thickness of the surface layer 2 and an application thereof free from discontinuities are obtained; moreover in this way, the surface layer 2 affects the whole surface E of device 1 with which the material of the surface layer 2 comes into contact.

The homogeneous thickness of the surface layer 2 may be obtained both if the outer surface E of device 1 is substantially linear and if device 1 comprises slits, grooves, recesses or other similar configurations.

The fact that the thickness of the surface layer 2 is homogeneous and that the latter affects the whole surface of device 1 with which it comes into contact allows a better distribution of the substances contained within the same surface layer 2 at a spatial level.

If the thickness and the arrangement of the surface layer 2 were not homogeneous, in fact, in some contact zones with the bone there may be the risk of an excessive concentration of substances contained in the surface layer 2 and in some others, a complete absence of the same. Moreover, thanks to the thickness homogeneity and to the application without discontinuity of the surface layer 2, zones of device 1 are prevented from being without surface coating 2. In this way, in fact, the material of device 1 is prevented from directly contacting the bony tissue since the prevention and/or healing of the infection in such zones would not take place. The application of the porous surface layer on the device does not change in any way the original usage technique. Therefore, no specific instrument is required for either the insertion within the bone or the subsequent removal.

At a later time, pores 2' cause the escape of drugs and/or medications from the surface layer 2.

In one version of the invention, at each subsequent immersion, device 1 or the intramedullary nail C is upturned for ensuring a better coverage evenness of the surface layer 2 on the same intramedullary nail. Each centimetre of intramedullary nail is coated with about 15-60 mg of surface layer 2 and preferably with 35 mg.

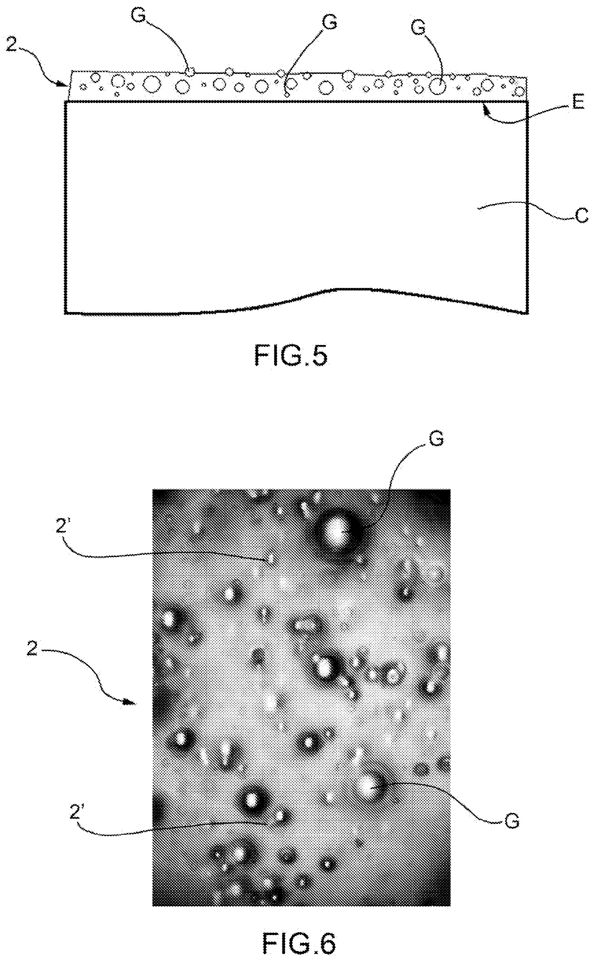

Figure 5 shows a schematic view of a version of the invention wherein the surface layer 2 is deposited through coating of a device 1 or an intramedullary nail C. Spherules of antibiotic G are visible within the surface layer 2. Figure 6 shows a view taken at the optical microscope of a version of the invention which depicts the surface layer 2 at a 20X magnification at a depth of 0.1 mm relative to the outer surface S, wherein pores 2' and the spherules of antibiotic G are visible.

The device according to the invention may be subject to several modifications and variations, all falling within the inventive concept expressed in the annexed claims. All the details may be replaced with other technically equivalent elements and the materials may be different according to the needs, without departing from the scope of the present invention. While the device has been described with particular reference to the annexed figures, the reference numerals used in the description and in the claims are used for improving the invention intelligibility and constitute no limitation to the claimed protection scope.

## Claims

1. A disposable temporary device (1) for prevention and/or treatment of infections of human limbs, comprising:
an outer surface (E),
a surface layer (2), with an external surface (S), said surface layer (2) comprising a biologically compatible material in a fluid solidifiable, wherein once the material the surface layer is made of has cured and solidified, the surface layer comprises a solid porous or spongy material, said surface layer (2) comprising pores (2') arbitrarily present in said external surface (S) or inside said surface layer (2), said pores (2') having a random disposal,
wherein
said surface layer (2) is strictly tight to and placed in contact with said outer surface (E) of said device (1) and has a continuous and homogeneous arrangement over said outer surface (E) whereon said surface layer (2) is adherent and in contact with and wherein said pores (2') have dimension less than 100 micron.

2. Device according to claim 1, wherein said material of said surface layer (2) is soluble or non soluble in the human body and is selected from the group comprising bone cements and/or reabsorbable or biodegradable bone cements, and/or a dispersion of polymers of polymethylmethacrilate (PMMA) and/or PMMA esters, such as butylmethacrilate, etc, and/or copolymers, such as the copolymer of PMMA and of polymethylstyrene, etc, in an organic solvent, for example, like methyl methacrylate (MMA), chloroform, or other organic solvent or water, and/or a dispersion of polymers comprising polyglycolic acid (PGA), polylactic acid (PLA), L polylactic acid, L-D polylactic acid, trimethylencarbonate, polyvinylpyrrolidone (PVP) derivates, polyvinylchloride (PVC), amide or cellulose derivates and/or their isomers or racemic forms, and copolymers thereof and an organic solvent or water.

3. Device according to claim 1, wherein said pores (2') are interconnected pores.

4. Device according to any one of the previous claims, wherein said surface layer (2) is intimately tight to said outer surface (E) of said device (1) by mechanical and/or chemical-physical adherence.

5. Device according to claim 4, wherein said mechanical adherence is due to roughness on said outer surface (E) of said device (1), for example such as undercuts and/or microcavities in which the material of said surface layer (2) settles.

6. Device according to claim 4, wherein said chemical-physical adherence is due to the induced attraction of weak molecular forces, such as Van der Waal forces, that are exerted between said surface layer (2) and said device (1).

7. Device according to any one of the previous claims, wherein said surface layer (2) has a variable thickness between 10 micron and 1000 micron.

8. Device according to any one of the previous claims, wherein said surface layer (2) has a thickness between 100 and 400 micron.

9. Device according to any one of the previous claims, wherein said device (1) is made in a relatively rigid metallic or non metallic material and/or it comprises an intramedullary nail (C).

10. Device according to any one of the previous claims, wherein said surface layer (2) comprises drugs and/or medications in dry form suitable for being mixed with said material of which said surface layer (2) is made of and/or in fluid form suitable for allow the impregnation of said surface layer (2) to be carried out preliminary or during the insertion in the stabilization site.

11. Device according to any one of the previous claims, wherein said device (1) comprises an handling tool (14) to facilitate the removal of said device.

12. Device according to any one of the previous claims, comprising a sterile blister packaging for containing said device (1).

13. Method for the realization of a temporary disposal device (1) according to claim 1 for prevention and/or treatment of infections of human limbs, comprising the following steps:
providing a device (1) suitable for stabilization of human limbs and provided with an outer surface (E);
arranging a solution comprising biologically compatible material in a fluid solidifiable form with an organic and/or watery solvent, applying said solution over said outer surface (E) of said device (1),
drying said device (1) at a temperature of about 70°C to completely remove said residual solvent through the evaporation thereof and/or the polymerization of said material, thus realizing a porous or spongy surface layer (2), that has an external surface (S) and comprising some pores (2') which are present in an arbitrary manner on said external surface (S) or inside said surface layer (2) and with a random arrangement, wherein said surface layer (2) strictly tights to said device (1), wherein said pores (2') have dimension less than 100 micron.

14. Method according to claim 13, wherein said applying step is realized through at least one immersion of said device (1) in said solution and/or through painting and/or spraying of said solution on said device (1).

15. Method according to one of claims from 13 to 14, comprising the following steps:
adding a drug or other medicament, such as antibiotic, to said solution with a concentration of about 5-65%(p/p) in said material, and/or
impregnating said surface layer (2), preliminary or during the insertion in the stabilization site, with drugs and/or other medicament for the treatment of the infections.

16. Method according to one of claims from 13 to 15, wherein said step of arranging a solution comprises said biologically compatible material at a concentration of about 1-75% (p/p) in the solvent.

17. Method according to one of claims from 13 to 16, comprising a homogenizing step of said solution before to the applying step.

18. Method according to one of claims from 13 to 17, comprising a step of waiting 10-60 minutes after said applying step.

19. Method according to one of claims from 13 to 18, comprising a step of repeating said applying step and said waiting step for at least two more times.

20. Method according to one of claims from 13 to 19, comprising a step of evaporating said solvent to eliminate most of the solvent itself.

21. Method according to one of claims from 13 to 20, wherein said drying step is done for about 5 hours.

22. Method according to claim 13, wherein said step of providing a device (1) comprises predisposing an intramedullary nail (C) substantially of a rod shape.

## Patentansprüche

1. Provisorische Einwegvorrichtung (1) zur Vorbeugung und/oder Behandlung von Infektionen von menschlichen Gliedern, umfassend:
eine äußere Fläche (E),
eine Oberflächenschicht (2) mit einer Außenfläche (S), wobei die Oberflächenschicht (2) ein biologisch kompatibles Material in einem verfestigbaren Fluid umfasst, wobei die Oberflächenschicht nach dem Aushärten und Verfestigen des Materials, aus dem die Oberflächenschicht hergestellt ist, ein festes poröses oder schwammartiges Material umfasst,
wobei die Oberflächenschicht (2) Poren (2') umfasst, die auf der Außenfläche (S) oder innerhalb der Oberflächenschicht (2) willkürlich vorhanden sind, wobei die Poren (2') eine willkürliche Anordnung aufweisen,
wobei die Oberflächenschicht (2) vollkommen dicht und in Kontakt mit der äußeren Fläche (E) der Vorrichtung (1) angeordnet ist und eine durchgehende, homogene Anordnung auf der äußeren Fläche (E) aufweist, auf der die Oberflächenschicht (2) haftet und sich in Kontakt befindet und wobei die Poren (2') eine Größe von weniger als 100 Mikron aufweisen.

2. Vorrichtung nach Anspruch 1, wobei das Material der Oberflächenschicht (2) löslich oder unlöslich in dem menschlichen Körper ist und ausgewählt ist aus der Gruppe umfassend Knochenzemente und/oder resorbierbare oder biologisch abbaubare Knochenzemente und/oder eine Dispersion von Polymeren von Polymethylmethacrylat (PMMA) und/oder PMMA-Estern wie Butylmethacrylat usw. und/oder Copolymeren wie PMMA-Copolymer und Polymethylstyrol-Copolymer usw. in einem organischen Lösemittel wie beispielsweise Methylmethacrylat (MMA), Chloroform oder einem anderen organischen Lösemittel oder Wasser und/oder eine Dispersion von Polymeren umfassend Polyglycolsäure (PGA), Polymilchsäure (PLA), Poly-L-Milchsäure, Poly-L-D-Milchsäure, Trimethylencarbonat, Polyvinylpyrrolidon (PVP) Derivate, Polyvinylchlorid (PVC), Amide oder Cellulosederivate und/oder deren Isomere oder racemische Formen und Copolymere davon und ein organisches Lösemittel oder Wasser.

3. Vorrichtung nach Anspruch 1, wobei die Poren (2') miteinander verbundene Poren sind.

4. Vorrichtung nach einem der vorstehenden Ansprüche, wobei die Oberflächenschicht (2) durch mechanische und/oder physikalisch-chemische Haftung ganz eng mit der äußeren Fläche (E) der Vorrichtung (1) verbunden ist.

5. Vorrichtung nach Anspruch 4, wobei die mechanische Haftung dank der Rauigkeit der äußeren Fläche (E) der Vorrichtung (1), beispielsweise Hinterschneidungen und/oder Mikrohohlräume zustande kommt, in denen das Material der Oberflächenschicht (2) sich absetzt.

6. Vorrichtung nach Anspruch 4, wobei die chemisch-physikalische Haftung dank der Anziehungskraft schwacher Molekularkräfte, wie Van-der-Waals-Kräfte, zustande kommt, die zwischen der Oberflächenschicht (2) und der Vorrichtung (1) ausgeübt sind.

7. Vorrichtung nach einem der vorstehenden Ansprüche, wobei die Oberflächenschicht (2) eine zwischen 10 Mikron und 1000 Mikron variierbare Dicke aufweist.

8. Vorrichtung nach einem der vorstehenden Ansprüche, wobei die Oberflächenschicht (2) eine Dicke zwischen 100 Mikron und 400 Mikron aufweist.

9. Vorrichtung nach einem der vorstehenden Ansprüche, wobei die Vorrichtung (1) aus einem relativ starren metallischen oder nicht-metallischen Material hergestellt ist und/oder einen Marknagel (C) umfasst.

10. Vorrichtung nach einem der vorstehenden Ansprüche, wobei die Oberflächenschicht (2) Medikamente und/oder Medikationen in trockener Form umfasst, geeignet zur Mischung mit dem Material, aus dem die Oberflächenschicht (2) hergestellt ist, und/oder in flüssiger Form, geeignet zur Durchtränkung der Oberflächenschicht (2), was vor oder während des Einsetzens in den Situs zur Stabilisierung ausgeführt wird.

11. Vorrichtung nach einem der vorstehenden Ansprüche, wobei die Vorrichtung (1) ein Handhabungswerkzeug (14) umfasst, um das Entfernen der Vorrichtung zu erleichtern.

12. Vorrichtung nach einem der vorstehenden Ansprüche, umfassend eine sterile Blisterverpackung zur Aufnahme der Vorrichtung (1).

13. Verfahren zur Ausführung einer provisorischen Einwegvorrichtung (1) nach Anspruch 1 zur Vorbeugung und/oder Behandlung von Infektionen menschlicher Glieder, umfassend folgende Schritte:
Bereitstellen einer Vorrichtung (1), die zur Stabilisierung von menschlichen Gliedern geeignet und mit einer äußeren Fläche (E) versehen ist,
Anordnen einer Lösung umfassend ein biologisch kompatibles Material in Form eines verfestigbaren Fluids mit einem organischen und/oder wässrigen Lösemittel, Auftragen der Lösung auf der äußeren Fläche (E) der Vorrichtung (1),
Trocknen der Vorrichtung (1) bei einer Temperatur von ca. 70 °C, um das restliche Lösemittel durch Verdunstung vollständig davon zu entfernen und/oder Polymerisierung des Materials und dadurch Herstellen einer porösen oder
schwammartigen Oberflächenschicht (2), die eine Außenfläche (S) aufweist und einige Poren (2') umfasst, die auf willkürliche Weise auf der Außenfläche (S) oder in der Oberflächenschicht (2) in willkürlicher Anordnung vorhanden sind, wobei die Oberflächenschicht (2) eng an der Vorrichtung (1) anliegt, wobei die Poren (2') eine Größe von weniger als 100 Mikron aufweisen.

14. Verfahren nach Anspruch 13, wobei der Schritt des Aufbringens durch mindestens ein Tauchbad der Vorrichtung (1) in die Lösung und/oder durch Bepinseln und/oder Aufsprühen der Lösung auf die Vorrichtung (1) durchgeführt wird.

15. Verfahren nach einem der Ansprüche 13 bis 14, umfassend folgende Schritte:
Zusetzen eines Medikaments oder anderer Heilmittel, beispielsweise Antibiotika, zu der Lösung in einer Konzentration von ca. 5 % - 65 % (p/p) in dem Material und/oder Tränken der Oberflächenschicht (2) - vor oder während des Einsetzens in den Situs zur Stabilisierung - mit Medikamenten und/oder anderen Heilmitteln zur Behandlung der Infektionen.

16. Verfahren nach einem der Ansprüche 13 bis 15, wobei der Schritt des Anordnens einer Lösung das biologisch kompatible Material in einer Konzentration von ca. 1 % - 75 % (p/p) in dem Lösemittel umfasst.

17. Verfahren nach einem der Ansprüche 13 bis 16, umfassend einen Schritt zur Homogenisierung der Lösung vor dem Schritt des Aufbringens.

18. Verfahren nach einem der Ansprüche 13 bis 17, umfassend einen Schritt des Wartens von 10 - 60 Minuten nach dem Schritt des Aufbringens.

19. Verfahren nach einem der Ansprüche 13 bis 18, umfassend einen Schritt des mindestens 2-maligen Wiederholens der Schritte des Aufbringens und des Wartens.

20. Verfahren nach einem der Ansprüche 13 bis 19, umfassend einen Schritt des Verdunstens des Lösemittels, um den größten Teil des Lösemittels zu beseitigen.

21. Verfahren nach einem der Ansprüche 13 bis 20, wobei der Schritt des Trocknens ca. 5 Stunden lang vorgenommen wird.

22. Verfahren nach Anspruch 13, wobei der Schritt des Bereitstellens einer Vorrichtung (1) das Vorsehen eines im Wesentlichen stabförmigen Marknagels (C) umfasst.

## Revendications

1. Dispositif temporaire jetable (1) pour la prévention et/ou le traitement d'infections de membres humains, comprenant :
une surface extérieure (E),
une couche de surface (2), avec une surface externe (S), ladite couche de surface (2) comprenant un matériau biologiquement compatible sous une forme fluide pouvant se solidifier, dans lequel, une fois que le matériau dont est constituée la couche de surface a durci et s'est solidifié, la couche de surface comprend un matériau spongieux ou poreux solide,
ladite couche de surface (2) comprenant des pores (2') présents arbitrairement dans ladite surface externe (S) ou à l'intérieur de ladite couche de surface (2), lesdits pores (2') ayant une disposition aléatoire,
dans lequel ladite couche de surface (2) est strictement serrée et mise en contact avec ladite surface extérieure (E) dudit dispositif (1) et présente une disposition continue et homogène sur ladite surface extérieure (E) sur laquelle est adhérente et avec laquelle est en contact ladite couche de surface (2) et dans lequel lesdits pores (2') ont une dimension inférieure à 100 microns.

2. Dispositif selon la revendication 1, dans lequel ledit matériau de ladite couche de surface (2) est soluble ou non soluble dans le corps humain et est sélectionné à partir du groupe comprenant des ciments osseux et/ou des ciments osseux résorbables ou biodégradables et/ou une dispersion de polymères de polyméthacrylate de méthyle (PMMA) et/ou d'esters de PMMA, tels que le méthacrilate de butyle, etc., et/ou de copolymères, tels que le copolymère de PMMA et de poly-méthylstyrène, etc., dans un solvant organique, par exemple, comme le méthacrylate de méthyle (MMA), le chloroforme, ou un autre solvant organique ou l'eau, et/ou une dispersion de polymères comprenant l'acide polyglycolique (PGA), l'acide polylactique (PLA), l'acide polylactique L, l'acide polylactique L-D, le carbonate de triméthylène, les dérivés de la polyvinylpyrrolidone (PVP), le poly(chlorure de vinyle) (PVC), les dérivés d'amide ou de cellulose et/ou leurs isomères ou formes racémiques, et leurs copolymères et un solvant organique ou l'eau.

3. Dispositif selon la revendication 1, dans lequel lesdits pores (2') sont des pores interconnectés.

4. Dispositif selon l'une quelconque des revendications précédentes, dans lequel ladite couche de surface (2) est étroitement serrée sur ladite surface extérieure (E) dudit dispositif (1) par adhérence mécanique et/ou physico-chimique.

5. Dispositif selon la revendication 4, dans lequel ladite adhérence mécanique est due à une rugosité sur ladite surface extérieure (E) dudit dispositif (1), par exemple telle que des entailles et/ou des microcavités dans lesquelles le matériau de ladite couche de surface (2) se dépose.

6. Dispositif selon la revendication 4, dans lequel ladite adhérence physico-chimique est due à l'attraction induite de forces moléculaires faibles, telles que des forces de Van der Waal, qui sont exercées entre ladite couche de surface (2) et ledit dispositif (1).

7. Dispositif selon l'une quelconque des revendications précédentes, dans lequel ladite couche de surface (2) a une épaisseur variable entre 10 microns et 1000 microns.

8. Dispositif selon l'une quelconque des revendications précédentes, dans lequel ladite couche de surface (2) a une épaisseur entre 100 et 400 microns.

9. Dispositif selon l'une quelconque des revendications précédentes, dans lequel ledit dispositif (1) est réalisé en un matériau métallique ou non métallique relativement rigide et/ou il comprend un clou intramédullaire (C).

10. Dispositif selon l'une quelconque des revendications précédentes, dans lequel ladite couche de surface (2) comprend des médicaments et/ou médications sous forme sèche adaptée pour être mélangée avec ledit matériau dont est constituée ladite couche de surface (2) et/ou sous forme fluide adaptée pour permettre d'effectuer l'imprégnation de ladite couche de surface (2) préalablement ou durant l'insertion dans le site de stabilisation.

11. Dispositif selon l'une quelconque des revendications précédentes, dans lequel ledit dispositif (1) comprend un outil de manipulation (14) pour faciliter l'enlèvement dudit dispositif.

12. Dispositif selon l'une quelconque des revendications précédentes, comprenant un emballage blister stérile pour contenir ledit dispositif (1).

13. Procédé pour la réalisation d'un dispositif temporaire jetable (1) selon la revendication 1 pour la prévention et/ou le traitement d'infections de membres humains, comprenant les étapes suivantes :
la fourniture d'un dispositif (1) adapté pour la stabilisation de membres humains et pourvu d'une surface extérieure (E),
la disposition d'une solution comprenant un matériau biologiquement compatible sous une forme fluide pouvant se solidifier avec un solvant aqueux et/ou organique, en appliquant ladite solution sur ladite surface extérieure (E) dudit dispositif (1),
le séchage dudit dispositif (1) à une température d'environ 70°C pour éliminer complètement ledit solvant résiduel par le biais de son évaporation et/ou la polymérisation dudit matériau, en réalisant ainsi une couche de surface poreuse ou spongieuse (2), qui a une surface externe (S) et comprenant des pores (2') qui sont présents d'une manière arbitraire sur ladite surface externe (S) ou à l'intérieur de ladite couche de surface (2) et avec une disposition aléatoire, dans lequel ladite couche de surface (2) est strictement serrée sur ledit dispositif (1), dans lequel lesdits pores (2') ont une dimension inférieure à 100 microns.

14. Procédé selon la revendication 13, dans lequel ladite étape d'application est réalisée par le biais d'au moins une immersion dudit dispositif (1) dans ladite solution et/ou par l'application à la brosse et/ou la vaporisation de ladite solution sur ledit dispositif (1).

15. Procédé selon une des revendications 13 à 14, comprenant les étapes suivantes :
l'ajout d'un médicament ou autre produit pharmaceutique, tel qu'un antibiotique, à ladite solution avec une concentration d'environ 5-65% (p/p) dans ledit matériau, et/ou l'imprégnation de ladite couche de surface (2), préalablement ou durant l'insertion dans le site de stabilisation, avec des médicaments et/ou autres médications pour le traitement des infections.

16. Procédé selon une des revendications 13 à 15, dans lequel l'étape de disposition d'une solution comprend ledit matériau biologiquement compatible avec une concentration d'environ 1-75% (p/p) dans le solvant.

17. Procédé selon une des revendications 13 à 16, comprenant une étape d'homogénéisation de ladite solution avant l'étape d'application.

18. Procédé selon une des revendications 13 à 17, comprenant une étape d'attente de 10-60 minutes après ladite étape d'application.

19. Procédé selon une des revendications 13 à 18, comprenant une étape de répétition, au moins deux fois de plus, de ladite étape d'application et de ladite étape d'attente.

20. Procédé selon une des revendications 13 à 19, comprenant une étape d'évaporation dudit solvant pour éliminer la plupart du solvant lui-même.

21. Procédé selon une des revendications 13 à 20, dans lequel ladite étape de séchage est effectuée pendant environ 5 heures.

22. Procédé selon la revendication 13, dans lequel ladite étape de fourniture d'un dispositif (1) comprend la prédisposition d'un clou intramédullaire (C) sensiblement en forme de tige.
